(19) **Europäisches Patentamt / European Patent Office / Office européen des brevets**

(11) **EP 2 405 767 B1**

(12) **FASCICULE DE BREVET EUROPEEN**

(45) Date de publication et mention
de la délivrance du brevet:
**11.11.2015 Bulletin 2015/46**

(21) Numéro de dépôt: **10710870.6**

(22) Date de dépôt: **09.03.2010**

(51) Int Cl.:
*A23K 1/00* *(2006.01)*      *A23L 1/00* *(2006.01)*
*A23L 1/30* *(2006.01)*

(86) Numéro de dépôt international:
**PCT/FR2010/000193**

(87) Numéro de publication internationale:
**WO 2010/103201 (16.09.2010 Gazette 2010/37)**

(54) **GRANULÉ PROBIOTIQUE STABLE ET SON PROCÉDÉ DE PRÉPARATION**

PROBIOTISCHES GRANULAT UND VERFAHREN ZUR HERSTELLUNG

PROBIOTIC GRANULE AND PROCESS OF MANUFACTURE

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **10.03.2009 FR 0901094**

(43) Date de publication de la demande:
**18.01.2012 Bulletin 2012/03**

(73) Titulaire: **Lesaffre et Compagnie**
**75001 Paris (FR)**

(72) Inventeurs:
• **HIOLLE, Amélie**
 **F-59144 Anfroipret (FR)**
• **SAMPSONIS, Cécile**
 **F-59910 Bondues (FR)**

• **AUCLAIR, Eric**
 **F-59710 Avelin (FR)**
• **LENOIR, Christian**
 **F-59520 Marquette Lez Lille (FR)**

(74) Mandataire: **Cabinet Plasseraud**
**52, rue de la Victoire**
**75440 Paris Cedex 09 (FR)**

(56) Documents cités:
**EP-A1- 0 636 692**     **WO-A1-97/16198**
**WO-A2-2007/135278**    **FR-A1- 2 909 685**
**US-A- 4 335 144**

• **"BIOSAF Product Information Sheet" INTERNET CITATION XP002428776 Extrait de l'Internet: URL:http://www.saf-agri.com/english/infobi o.htm> [extrait le 2007-04-18]**

**Description**

La présente invention se rapporte au domaine des compositions probiotiques utiles pour l'alimentation et la santé humaine et/ou animale. Elle se rapporte particulièrement à un procédé de granulation associé à une composition comprenant des microorganismes probiotiques permettant l'obtention de granulés probiotiques stables.

Le terme « probiotiques » désigne des micro-organismes vivants ou revivifiables, qui lorsqu'ils sont ingérés en quantité suffisante exercent un effet positif sur l'organisme hôte en améliorant les propriétés de sa flore intestinale au-delà des effets nutritionnels traditionnels. Ils constituent une alternative à l'emploi des antibiotiques dans l'alimentation animale. Il s'agit le plus souvent de bactéries ou de levures présentes soit dans les aliments soit dans les compléments alimentaires.

Il existe quatre grands types de probiotiques :

- les ferments lactiques (les lactobacilles et les coques),

- les bifidobactéries d'origine humaine ou animale,

- les différentes levures dont les levures de type *Saccharomyces*,

- les autres bactéries sporulées, dont *Bacillus subtillis* et *cereus*.

Les micro-organismes tués par la chaleur ne répondent pas à la définition des probiotiques, même si certains effets thérapeutiques ont pu leur être attribués.

Par extension, le terme « probiotiques » désigne également les aliments contenant de tels micro-organismes.

Les probiotiques sont de plus en plus utilisés tant en alimentation humaine qu'en alimentation animale, notamment comme substituts aux antibiotiques compte tenu notamment d'un retour à une alimentation plus saine, plus naturelle et plus respectueuse de l'environnement. En nutrition animale, ces micro-organismes sont en général ajoutés dans un aliment granulé par l'intermédiaire d'un pré-mélange contenant également des vitamines, des oligo-éléments et des acides aminés.

De manière générale, la fabrication des granulés est divisée en au moins deux étapes :

A- la préparation d'un pré-mélange d'additifs,
B- la production par le fabricant d'aliment de granulés comprenant le pré-mélange (A).

Les microorganismes probiotiques sont incorporés soit dans le pré-mélange soit lors de la fabrication des granulés.

L'introduction des micro-organismes probiotiques dans les granulés n'est cependant pas aisée. Les micro-organismes probiotiques sont le plus souvent détruits au cours du processus de granulation. En effet, la fabrication des granulés est effectuée en présence de vapeur à des températures comprises entre 60°C et 120°C et sous des pressions allant de 0,5 à 40 bars. Ces procédés de granulation associent des contraintes thermiques, mécaniques et d'humidité très importantes auxquelles les microorganismes doivent survivre.

Dans la demande de brevet EP 0 694 610, la résistance à la granulation de levures de l'espèce *Phaffia rhodozyma* qui produisent un colorant, l'astaxanthine, et sont utilisées dans l'alimentation piscicole pour donner la couleur rouge aux saumons et truites d'élevage, a été améliorée par séchage en présence d'un sucre comme le glucose.

Cependant, il ne s'agissait pas là de levures probiotiques qui doivent rester vivantes jusqu'à leur ingestion par les animaux afin de remplir leurs fonctions.

Dans la demande de brevet n° WO 2007/135278, on a décrit que des micro-organismes probiotiques pouvaient présenter une stabilité améliorée dans les pré-mélanges au cours du temps par mélange avec des parois de levures et/ou des levures désactivées en lieu et place des autres supports de pré-mélange traditionnellement utilisés tels la farine de blé ou le carbonate de calcium, cette stabilité améliorée au cours du temps les rendant alors plus résistants à la granulation.

Il existe donc un réel besoin en micro-organismes probiotiques qui soient à la fois stables au cours du temps et aussi au cours du processus de granulation de telle sorte que l'effet escompté sur la santé de l'être qui les ingère soit réel.

La demanderesse a trouvé que la sélection d'un microorganisme probiotique dont la définition exacte des caractéristiques associée à l'optimisation des conditions opératoires du procédé de granulation permet de résoudre le problème cité précédemment.

La solution de l'invention permet une meilleure stabilité des microorganismes, c'est-à-dire des pertes après traitement inférieures à 1logCFU/g (CFU : Colony Forming Unit).

Plus spécifiquement, la demanderesse a trouvé que la solution à ce problème réside dans la combinaison des deux éléments suivants :

- tout d'abord, la sélection d'un microorganisme choisi parmi les levures probiotiques présentées sous une forme sèche agglomérée en sphérules ayant un taux de matière sèche (MS) compris entre 93 et 98 % et un diamètre médian des sphérules (d) compris entre 500 et 1500µm, et

- ensuite le choix précis des conditions opératoires du procédé de granulation mis en oeuvre pour la préparation des granulés destinés aux applications finales.

La solution à la base de la présente invention ne génère pas des pertes en microorganismes probiotiques après granulation supérieures à 1 log CFU/g d'aliment granulé comme expliqué plus loin.

Les sphérules selon l'invention présentent avantageusement un taux de matière sèche compris entre 93 et 96% et un diamètre médian (d) compris entre 800 et 1200µm.

De manière générale, la chaîne de fabrication des granulés est divisée en plusieurs étapes :

**A- la préparation d'un pré-mélange d'additifs** (vitamines, oligo-éléments, micro-organismes, huiles essentielles ... dilués sur un support ou adjuvant. Il existe plusieurs adjuvants, d'origine minérale ou végétale, utilisés classiquement comme la farine à base de blé, le carbonate de calcium, l'argile sépiolitique ou les drèches d'amidonnerie). Le pré-mélange se présente sous une forme farine finement broyée (diamètre médian des particules de 100µm environ).

**B- la production des granulés par le fabricant d'aliment comprenant :**

a- une phase de macro-mélange avec des matières premières broyées (blé, maïs, orge, tourteaux, ...), des additifs et des prémélanges. Ce macro-mélange se présente sous forme d'une farine grossière (diamètre médian des particules de 500µm),
b- une phase de pré-granulation (traitement thermique à la vapeur seule ou avec contraintes mécaniques),
c- une phase de granulation (fabrication des granulés) caractérisée par une température de la filière (T) et un diamètre des granulés (D),
d- une phase de séchage-refroidissement.

Généralement, les levures sont incorporées dans le pré-mélange (étape A), ou directement au moment du macro-mélange (étape Ba).

L'invention a pour objet un procédé de préparation de granulés probiotiques consistant en les étapes suivantes :

- incorporation à un mélange nutritionnel adapté à l'application visée, d'une composition probiotique (A) consistant en :

  - de 2 à 30 % en poids du poids total de la composition, de levure probiotique définie plus loin ayant un taux de matière sèche (MS) compris entre 93 et 98% et un diamètre médian des sphérules (d) compris entre 800 et 1200µm, et
  - de 70 à 98 % en poids du poids total de la composition, d'un complément nutritionnel comprenant au moins un constituant choisi parmi les vitamines, les oligoéléments, les acides aminés, et autres additifs destinés à l'alimentation et/ou la santé animale ou humaine,

- injection de vapeur d'eau à une température comprise entre 60°C et 85°C sous des pressions allant de 0,5 à 4 bars dans le mélange obtenu à l'étape précédente,
- granulation par pressage à une température comprise entre 70 et 92°C pour l'obtention de granulés de diamètre (D) compris entre 2 et 6 mm, et
- refroidissement.

La température (T) de la filière de granulation, le diamètre des granulés (D), le taux de matière sèche (MS), et le diamètre médian des sphérules (d) sont fixés de manière à satisfaire l'équation suivante :

$$X = -196,482 - 0,023\ d + 2,256\ (MS) - 14,793\ T + 3,046\ D + 6,25.10^{-5}\ d\ (MS) + 0,001\ d.T + 5,63\,10^{-5}\ d.T$$
$$+ 0,167(MS)\,.T - 0,036\ (MS)\,.T - 0,023D.T + 4,06.10^{-6}\ d^2 + 0,003\ T^2$$

dans laquelle les pertes en levure après granulation X (logCFU/g initiales - logCFU/g après granulation) sont inférieures à 1 log CFU/g d'aliment granulé.

Selon un mode de réalisation préférentiel le procédé de l'invention est mis en oeuvre dans les conditions suivantes :

| Température filière (°C) | Diamètre des granulés (mm) | Diamètre des sphérules (μm) | Matière sèche des sphérules (%) |
|---|---|---|---|
| 85 | 3,5 | 1000 | 94,0 |
|  |  | 1200 | 93,0 |
| 90 | 4 | 800 | 94,0 |
|  |  | 970 | 94,8 |
| 92 | 4 | 865 | 94,8 |
| 92 | 4 | 950 | 94,7 |

La composition probiotique de l'invention comprend préférentiellement :

- de 5 à 20 % en poids du poids total de la composition de levure probiotique,
- de 80 à 95 % en poids du poids total de la composition, d'un complément nutritionnel comprenant au moins un constituant choisi parmi les vitamines, les oligoéléments, les acides aminés, et autres additifs destinés à l'alimentation et/ou la santé animale ou humaine.

La préparation et le séchage des levures est une technique bien connue. On cultive la levure sous forme d'une biomasse pure, la levure est ensuite récoltée, mélangée éventuellement avec des auxiliaires technologiques à raison de quelques pour cent de la matière sèche mise en oeuvre, extrudée et séchée par différentes techniques : fluidisation, séchage sur bande ou encore en tambour rotatif. Ces différentes techniques sont décrites dans des manuels de base comme Yeast Technology, Reed and Nagodawithana, second édition, an AVI book, Van Nostrand Reinhold. L'homme du métier sait adapter les conditions opératoires notamment les dimensions de la filière de granulation et des tamis, ainsi que les conditions de séchage pour obtenir les levures selon la présente invention comme indiqué dans la partie expérimentale.

Les levures probiotiques sont choisies parmi les levures correspondant aux souches choisies dans le groupe comprenant la souche *Saccharomyces cerevisiae* Sc47 déposée auprès du NCYC sous le numéro 47, la souche *Saccharomyces cerevisiae* déposée auprès de la collection Pasteur (CNCM) sous le numéro I-1077, la souche *Saccharomyces cerevisiae* déposée auprès de la collection Pasteur (CNCM) sous le numéro I-1079, la souche *Saccharomyces cerevisiae* déposée auprès de la collection MUCL sous le numéro 39 885, la souche *Saccharomyces cerevisiae* déposée auprès de la collection CBS sous le numéro 39 493.94, la souche *Kluyveromyces marxianus* déposée auprès de la collection MUCL sous le numéro 39434, et leurs mélanges.

De façon tout à fait préférentielle la composition probiotique selon la présente invention comprend des levures correspondant à la souche *Saccharomyces cerevisiae* Sc47 déposée auprès du NCYC sous le numéro *47*.

La composition probiotique de l'invention peut être utilisée dans l'alimentation animale et/ou humaine. Elle peut être administrée sous différentes formes.

Selon un mode préféré de l'invention, la composition probiotique destinée à l'alimentation animale est administrée sous la forme de granulé.

Selon un mode de réalisation préféré de l'invention, les conditions suivantes sont appliquées :

| Température filière (°C) | Diamètre des granulés (mm) | Diamètre des sphérules (μm) | Matière sèche des sphérules (%) |
|---|---|---|---|
| 85 | 3,5 | 1000 | 94,0 |
|  |  | 1200 | 93,0 |
| 90 | 4 | 800 | 94,0 |
|  |  | 970 | 94,8 |
| 92 | 4 | 865 | 94,8 |
|  |  | 950 | 94,7 |

Les conditions suivantes sont celles qui provoquent les pertes les plus faibles :

| Température filière (°C) | Diamètre des granulés (mm) | Diamètre des sphérules (µm) | Matière sèche des sphérules (%) |
|---|---|---|---|
| 92 | 4 | 865 | 94,8 |
| 92 | 4 | 950 | 94,7 |

La composition (A) est introduite dans les granulés à raison de 0,01 à 10% de levure probiotique selon l'invention par rapport au poids sec de l'aliment, ce qui se traduit par 1.E7 à 1.E12 CFU par kg d'aliments granulés.

Le procédé de l'invention permet d'obtenir un granulé comprenant de 0,01 à 10 % de levure probiotique et de 90 à 99,9% en poids d'un mélange nutritionnel adapté.

Selon une forme préférentielle le granulé comprend de 0,01% à 5 % de levure probiotique et de 95 à 99,9% du mélange nutritionnel.

Le granulé est obtenu avec des pertes des microorganismes probiotiques après granulation inférieures à 1log CFU/g d'aliment granulé.

Le granulé probiotique obtenu par le procédé de l'invention peut être utilisé en alimentation animale et/ou humaine.

Ce qui suit décrit les modes de réalisation de l'invention sans en limiter la portée.

Les études suivantes ont été réalisées :

- identification des paramètres de granulation critiques pour les levures,
- étude des effets individuels des paramètres identifiés sur la survie de la levure en fonction de ses propriétés physico-chimiques,
- étude de la granulation complète sur la levure (en pilote) dans le but d'établir un modèle mathématique reliant les propriétés physiques des sphérules et leur stabilité,
- détermination d'un couple [granulométrie et MS] conduisant à des pertes minimales,
- validation du couple [granulométrie et MS des sphérules] au cours de granulations industrielles.

Dans ce travail, la levure sous forme ADY/SPH a été utilisée. C'est une levure sèche de panification à hydrater. Elle nécessite une réhydratation avant usage. Elle se présente sous forme de sphérules.

### I. Identification des paramètres de granulation critiques pour les levures

Des plans expérimentaux complexes ont permis d'obtenir la classification suivante des paramètres critiques par ordre décroissant de sévérité : diamètre des granulés, température de pré-granulation, longueur des canaux de la filière, humidité de l'aliment. Les contraintes physiques sont donc l'humidité de l'aliment, la température de traitement et la compression (diamètre et longueur des canaux étant directement reliés aux forces de compression).

### II. Effet de la granulation complète sur la stabilité de levures ADY/SPH

Un plan expérimental complet (multi-factoriel, multi-niveaux) faisant varier les paramètres ci-dessous a été suivi :

- paramètres liés à la levure : matière sèche et diamètre médian
- paramètres de la granulation : température de filière, diamètre des granulés.

Les essais ont été réalisés sur une presse pilote Kahl L14-175, 3kW.

| Facteurs | Niveaux | Valeurs | | | |
|---|---|---|---|---|---|
| Matière sèche (%) | 2 | 95 | 93 | | |
| Diamètre des sphérules (µm) | 4 | 1250 | 1050 | 865 | 570 |
| Température (°C) | 3 | 93 | 88 | 83 | |
| Diamètre granulés (mm) | 2 | 4 | 3,2 | | |

Pour exemple, voici quelques résultats obtenus :

| Diamètre médian des sphérules | MS | Diamètre des granulés | T°C | PERTES (logCFU/g) |
|---|---|---|---|---|
| 865 | 93 | 3,2 | 83 | 0,6 |
| 1250 | 95 | 3,2 | 83 | 0,5 |
| 1250 | 93 | 4 | 83 | 0,4 |
| 865 | 93 | 4 | 83 | 0,6 |
| 570 | 95 | 3,2 | 83 | 0,4 |
| 865 | 93 | 3,2 | 88 | 1,4 |
| 1250 | 95 | 3,2 | 93 | 1 |
| 1050 | 93 | 4 | 88 | 0,7 |
| 570 | 93 | 3,2 | 88 | 1,5 |
| 1250 | 95 | 3,2 | 88 | 1 |
| 1250 | 93 | 4 | 93 | 1,4 |
| 1050 | 95 | 3,2 | 93 | 0,6 |

Ce plan a conduit à l'établissement d'un modèle expliquant les pertes en levures après granulation en fonction de ces 4 facteurs.

L'équation mathématique du modèle complet est présentée ci-dessous (valable dans notre champ expérimental).

$$X = -196{,}482 - 0{,}023\ d + 2{,}256\ (MS) - 14{,}793\ T + 3{,}046\ D + 6{,}25.10^{-5}\ d\ (MS) + 0{,}001\ d.T + 5{,}63 10^{-5}\ d.T$$
$$+ 0{,}167(MS)\ .T - 0{,}036\ (MS)\ .T - 0{,}023 D.T + 4{,}06.10^{-6}\ d^2 + 0{,}003\ T^2$$

Avec:

X représente les pertes en microorganisme après granulation

$$(logCFU/g) = logCFU/g\ initiales - logCFU/g$$

d ($\mu$m) = le diamètre médian des sphérules
MS (%) = la matière sèche des sphérules
D (mm) = le diamètre des granulés
T (°C) = la température de la filière

Selon nos objectifs de stabilité - pertes inférieures à 1logCFU/g - les couples [MSxDiamètre médian des sphérules] qui nous intéressent, doivent être définis en fonction des conditions de granulation appliquées. L'équation mathématique devient alors :

$$1{,}0 > -196{,}482 - 0{,}023\ d + 2{,}256\ (MS) - 14{,}793\ T + 3{,}046\ D + 6{,}25.10^{-5}\ d\ (MS) + 0{,}001\ d.T +$$
$$5{,}63 10^{-5}\ d\ .T + 0{,}167(MS)\ .T - 0{,}036\ (MS)\ .T - 0{,}023 D.T + 4{,}06.10^{-6}\ d^2 + 0{,}003\ T^2$$

Il est important de noter que pour des conditions de granulation données, il existe une multitude de couples [MSxDiamètre médian des sphérules].

Voici quelques exemples de couples permettant d'avoir des pertes inférieures à 1,0logCFU/g d'aliment :

| Température filière (°C) | Diamètre des granulés (mm) | Diamètre des sphérules (μm) | Matière sèche des sphérules (%) |
|---|---|---|---|
| 85 | 3,5 | 1000 | 94,0 |
| | | 1200 | 93,0 |
| 90 | 4 | 800 | 94,0 |
| | | 970 | 94,8 |
| 92 | 4 | 865 | 94,8 |
| | | 950 | 94,7 |

Deux couples ont alors été retenus pour validation en granulation industrielle (selon les faisabilités de fabrication des produits) :

Couple 1 : d = 950 μm et MS = 94,7%
Couple 2 : d = 865 μm et MS = 94,8%

**Description détaillée du procédé de préparation de la levure selon l'invention**

La levure est préparée sous forme d'une biomasse pure, elle est ensuite récoltée, mélangée avec des auxiliaires technologiques à raison de quelques pour cent de la matière sèche mise en oeuvre, extrudée et séchée par différentes techniques : fluidisation, séchage sur bande ou encore en tambour rotatif.

**A - le diamètre médian (d) :**

Une maille d'extrusion de 2mm est utilisée dans le but d'obtenir des sphérules ayant un diamètre médian de 950 μm et une maille de 1.8mm afin d'avoir des sphérules ayant un diamètre médian de 865 μm.

**B - Le taux de matière sèche (MS) :**

Le procédé consiste en un séchage en tambour classique tel que décrit plus loin. Les temps de séchage ont été optimisés de manière à avoir les taux de MS désirés.

Description du procédé de fabrication

Nous distinguons plusieurs étapes de fabrication, une étape de déshydratation et une étape de séchage.

**A - la déshydratation**

Cette étape permet d'obtenir une pâte de levure à 33% (plus ou moins 3%) de MS à partir d'une crème de levure à 18% (plus ou moins 2%) de MS.

Principe :

Est incorporée à la crème de levure une saumure (eau + Na Cl) jusqu'à l'obtention d'une conductivité variant entre 15000 μS et 20000 μS.
Cette crème salée passe ensuite sur un deshydrateur (filtre rotatif sous vide) sur lequel nous aurons au préalable réalisé une précouche de fécule (eau + fécule). Après le passage sur le deshydrateur, la crème de levure passe d'un état liquide à un état de pâteux (30 à 36% de MS).
Cette pâte de levure est ensuite émiettée dans un granulateur (cylindre avec des bras mélangeurs brassant la levure et la poussant vers un autre cylindre au bout duquel se trouve une grille perforée à 2mm ou 1.8mm). Cette étape est primordiale dans l'obtention de la granulométrie souhaitée.
La levure émiettée est ensuite transportée vers les organes de séchage.

**B- Le séchage**

Le séchage se fait de manière discontinue dans un cylindre rotatif horizontal muni de pâles brassant la levure et à l'intérieur duquel passe un courant d'air chaud. La température et le débit de l'air chaud sont fixés de manière à atteindre une température levure de l'ordre de 43°C (+/- 1°C) en fin de séchage.
Une fois que la MS souhaitée est atteinte, la levure est vidangée vers un silo d'emballage.

**Revendications**

**1.** Procédé de granulation consistant en les étapes suivantes :

a - incorporation à un mélange nutritionnel adapté à l'application visée, d'une composition probiotique (A) consistant en :

- de 2 à 30 % en poids du poids total de la composition, de levure probiotique sous une forme sèche agglomérée en sphérules ayant un taux de matière sèche (MS) compris entre 93 et 98% et un diamètre médian des sphérules (d) compris entre 800 et 1200$\mu$m, et
- de 70 à 98 % en poids du poids total de la composition, d'un complément nutritionnel comprenant au moins un constituant choisi parmi les vitamines, les oligoéléments, les acides aminés, et autres additifs destinés à l'alimentation et/ou la santé animale ou humaine,

b - injection de vapeur d'eau à une température comprise entre 60°C et 85°C sous des pressions allant de 0,5 à 4 bars dans le mélange obtenu à l'étape précédente,
c - granulation par pressage à une température comprise entre 70°C et 92°C pour l'obtention de granulés de diamètre (D) compris entre 2 et 6 mm, et
d - refroidissement,
**caractérisé en ce que** la température (T) de la filière de granulation, le diamètre des granulés (D), le taux de matière sèche (MS), et le diamètre médian des sphérules (d) sont fixés de manière à satisfaire l'équation suivante :

$$\mathbf{X} = -196{,}482 - 0{,}023\ \mathbf{d} + 2{,}256\ (\mathbf{MS}) - 14{,}793\ \ \mathbf{T} + 3{,}046\ \mathbf{D} + 6{,}25.10^{-5}\ \mathbf{d}\ (\mathbf{MS}) +$$

$$0{,}001\ \mathbf{d.T} + 5{,}63.10^{-5}\ \mathbf{d.T} + 0{,}167(\mathbf{MS}).\mathbf{T} - 0{,}036\ (\mathbf{MS}).\mathbf{T} - 0{,}023\mathbf{D.T} + 4{,}06.10^{-6}\ \mathbf{d^2} +$$

$$0{,}003\ \mathbf{T^2}$$

dans laquelle les pertes en levure après granulation X (logCFU/g initiales - logCFU/g après granulation) sont inférieures à 1 log CFU/g d'aliment granulé,
et dans laquelle la levure probiotique est choisie parmi les levures correspondant aux souches choisies dans le groupe comprenant la souche *Saccharomyces cerevisiae* Sc47 déposée auprès du NCYC sous le numéro 47, la souche *Saccharomyces cerevisiae* déposée auprès de la collection Pasteur (CNCM) sous le numéro I-1077, la souche *Saccharomyces cerevisiae* déposée auprès de la collection Pasteur (CNCM) sous le numéro I-1079, la souche *Saccharomyces cerevisiae* déposée auprès de la collection MUCL sous le numéro 39 885, la souche *Saccharomyces cerevisiae* déposée auprès de la collection CBS sous le numéro 39 493.94, la souche *Kluyveromyces marxianus* déposée auprès de la collection MUCL sous le numéro 39434, et leurs mélanges.

**2.** Procédé de granulation selon la revendication 1 **caractérisé en ce qu'**il est mis en oeuvre dans l'une des conditions suivantes :

| Température filière (°C) | Diamètre des granulés (mm) | Diamètre des sphérules ($\mu$m) | Matière sèche des sphérules (%) |
|---|---|---|---|
| 85 | 3,5 | 1000 | 94,0 |
| | | 1200 | 93,0 |

(suite)

| Température filière (°C) | Diamètre des granulés (mm) | Diamètre des sphérules (μm) | Matière sèche des sphérules (%) |
|---|---|---|---|
| 90 | 4 | 800 | 94,0 |
| | | 970 | 94,8 |
| 92 | 4 | 865 | 94,8 |
| 92 | 4 | 950 | 94,7 |

3. Procédé de granulation selon l'une des revendications précédentes **caractérisé en ce que** la composition probiotique A consiste de préférence en :

- de 5 à 20 % en poids du poids total de la composition de levure probiotique,
- de 80 à 95 % en poids du poids total de la composition, d'un complément nutritionnel comprenant au moins un constituant choisi parmi les vitamines, les oligoéléments, les acides aminés, et autres additifs destinés à l'alimentation et/ou la santé animale ou humaine.

4. Procédé de granulation selon l'une quelconque des revendications 1 à 3 **caractérisé en ce que** la dite levure correspond à la souche *Saccharomyces cerevisiae* Sc47 déposée auprès du NCYC sous le numéro 47.

**Patentansprüche**

1. Verfahren zur Granulierung, bestehend aus den folgenden Schritten:

a- Beimischen einer probiotischen Zusammensetzung (A) in eine Nährmischung, die für die beabsichtigte Anwendung geeignet ist, bestehend aus:

- von 2 bis 30 Gewichts-%, bezogen auf das Gesamtgewicht der Zusammensetzung, von probiotischer Hefe in trockener, in Kügelchen agglomerierter Form mit einem Trockensubstanzgehalt (MS), der zwischen 93 und 98% umfasst ist, und einem mittleren Durchmesser der Kügelchen (d), der zwischen 800 und 1200 μm umfasst ist, und
- von 70 bis 98 Gewichts-%, bezogen auf das Gesamtgewicht der Zusammensetzung, von einem Nahrungsergänzungsmittel, umfassend mindestens eine Komponente, ausgewählt aus Vitaminen, Spurenelementen, Aminosäuren und anderen Additiven zur Verwendung in Lebensmitteln und/oder für die tierische oder menschliche Gesundheit,

b- Injizieren von Wasserdampf bei einer Temperatur, welche zwischen 60 °C und 85 °C umfasst ist, bei Drücken im Bereich von 0,5 bis 4 Bar in der in dem vorherigen Schritt erhaltenen Mischung,
c- Granulieren durch Pressen bei einer Temperatur, welche zwischen 70 °C und 92 °C umfasst ist, um ein Granulat mit einem Durchmesser (D), welcher zwischen 2 und 6 mm umfasst ist, zu erhalten, und
d- Kühlen,

**dadurch gekennzeichnet, dass** die Temperatur (T) der Granulierungspressform, der Durchmesser der Granulate (D), der Gehalt an Trockenmasse (MS) und der mittlere Durchmesser der Kügelchen (d) so eingestellt sind, dass sie die folgende Gleichung erfüllen:

$$X = -196,482 - 0,023\, d + 2,256\, (MS) - 14,793\, T + 3,046\, D + 6,25.10^{5}\, d\, (MS) + 0,001\ d.T + 5,63.10^{-5}\ d.T + 0,167\ (MS).T - 0,036\ (MS).T - 0,023\ D.T + 4,06.10^{-6}\ d^{2} + 0,003\ T^{2}$$

wobei die Hefeverluste nach der Granulierung X (logCFU/g Anfang - logCFU/g nach der Granululierung) weniger als 1 log CFU/g Granulatfutter sind,

und wobei die probiotische Hefe ausgewählt ist aus den Hefen entsprechend der Gattung ausgewählt aus der Gruppe umfassend die Gattung *Saccharomyces cerevisiae* Sc47 hinterlegt bei der NCYC unter der Nummer 47, die Gattung *Saccharomyces cerevisiae* hinterlegt bei der Collection Pasteur (CNCM) unter der Nummer I-1077, die Gattung *Saccharomyces cerevisiae* hinterlegt bei der Collection Pasteur (CNCM) unter der Nummer I-1079, die Gattung *Saccharomyces cerevisiae* hinterlegt bei der Collection MUCL unter der Nummer 39 885, die Gattung *Saccharomyces cerevisiae* hinterlegt bei der Collection CBS unter der Nummer 39 493.94, die Gattung *Kluyveromyces marxianus* hinterlegt bei der Collection MUCL unter der Nummer 39434, und Mischungen davon.

**2.** Verfahren zur Granulierung nach Anspruch 1, **dadurch gekennzeichnet, dass** es unter den folgenden Bedingungen umgesetzt wird :

| Granulierlochplatte-Temperatur (°C) | Durchmesser der Granulate (mm) | Durchmesser der Kügelchen (mm) | Trockenmasse der Kügelchen (%) |
|---|---|---|---|
| 85 | 3,5 | 1000 | 94,0 |
| | | 1200 | 93,0 |
| 90 | 4 | 800 | 94,0 |
| | | 970 | 94,8 |
| 92 | 4 | 865 | 94,8 |
| 92 | 4 | 950 | 94,7 |

**3.** Verfahren zur Granulierung nach einem der vorhergehenden Ansprüche, **dadurch gekennzeichnet, dass** die probiotische Zusammensetzung A vorzugsweise besteht aus :

- von 5 bis 20 Gewichts-%, bezogen auf das Gesamtgewicht der Zusammensetzung von probiotischer Hefe,
- von 80 bis 95 Gewichts-%, bezogen auf das Gesamtgewicht der Zusammensetzung, von einem Nahrungsergänzungsmittel, umfassend mindestens eine Komponente, ausgewählt aus Vitaminen, Spurenelementen, Aminosäuren und anderen Additiven zur Verwendung in Lebensmitteln und/oder für die tierische oder menschliche Gesundheit.

**4.** Verfahren zur Granulierung nach einem der Ansprüche 1 bis 3, **dadurch gekennzeichnet, dass** die Hefe der Gattung *Saccharomyces cerevisiae* Sc47 hinterlegt bei der NCYC unter der Nummer 47 entspricht.

## Claims

**1.** Granulation method consisting of the following steps:

a -incorporating, into a nutritional mixture adapted to the target application, a probiotic composition (A) consisting of:

- 2 to 30% by weight of the total weight of the composition, of probiotic yeast in a dry form agglomerated into spherules having content of dry matter equal to between 93 and 98% and a median spherule diameter (d) equal to between 800 and 1200$\mu$m, and
- from 70 to 98% by weight of the total weight of the composition, of a nutritional complement comprising at least one constituent chosen from vitamins, trace elements, amino acids and other additives intended for animal or human food and/or health.

b -injecting steam at a temperature of between 60°C and 85°C at pressures varying from 0.5 to 4 bars in the mixture obtained in the previous step,
c -granulating by pressing at a temperature of between 70°C and 92°C to obtain granules of large diameter granules (D) of between 2 and 6 mm, and
d -cooling,

**characterised in that** the temperature (T) of the granulation press, the diameter (D) of the granules, the content of dry matter (MS) and the median diameter (d) of the spherules are defined to satisfy the following equation:

$$X = -196.482 - 0.023\ d + 2.256\ (MS) - 14.793\ T + 3.046\ D + 6.25 \times 10^{-5}\ d\ (MS) + 0.001\ d.T + 5.63 \times 10^{-5}\ d.T + 0.167\ (MS).T - 0.036\ (MS).T - 0.023\ D.T + 4.06 \times 10^{-6}\ d^2 + 0.003\ T^2$$

in which yeast losses after granulation X (logCFU/g initial - logCFU/g after granulation) are less than 1 log CFU/g of granulated food,

and in which the probiotic yeast is chosen from yeasts corresponding to those chosen from the group comprising the *Saccharomyces cerevisiae* strain Sc47 deposited at the NCYC under number 47, the *Saccharomyces cerevisiae* strain deposited at the Pasteur collection (CNCM) under number 1-1077, the *Saccharomyces cerevisiae* strain deposited at the Pasteur collection (CNCM) under number 1-1079, the *Saccharomyces cerevisiae* strain deposited at the MUCL collection under number 39 885, the *Saccharomyces cerevisiae* strain deposited at the CBS collection under number 39 493.94, the *Kluyveromyces marxianus* strain deposited at the MUCL collection under number 39434 and mixtures thereof.

2. Granulation method according to claim 1, **characterised in that** it is used under one of the following conditions:

| Press temperature (°C) | Granule diameter (mm) | Spherule diameter ($\mu$m) | Dry matter in spherules (%) |
|---|---|---|---|
| 85 | 3.5 | 1000 | 94.0 |
| | | 1200 | 93.0 |
| 90 | 4 | 800 | 94.0 |
| | | 970 | 94.8 |
| 92 | 4 | 865 | 94.8 |
| 92 | 4 | 950 | 94.7 |

3. Granulation method according to one of the previous claims, **characterised in that** the probiotic composition A preferably consists of:

- 5 to 20% by weight of the total weight of the composition of the probiotic yeast,
- from 80 to 95% by weight of the total weight of the composition, of a nutritional complement comprising at least one constituent chosen from vitamins, trace elements, amino acids and other additives intended for animal or human food and/or health.

4. Granulation method according to any one of claims 1 to 3, **characterised in that** said yeast corresponds to *Saccharomyces cerevisiae* strain Sc47 deposited at the NCYC under number 47.

**RÉFÉRENCES CITÉES DANS LA DESCRIPTION**

**Documents brevets cités dans la description**

- EP 0694610 A
- WO 2007135278 A